(19) Europäisches Patentamt
European Patent Office
Office européen des brevets

(11) **EP 3 577 626 B1**

(12) **EUROPÄISCHE PATENTSCHRIFT**

(45) Veröffentlichungstag und Bekanntmachung des Hinweises auf die Patenterteilung:
**27.07.2022 Patentblatt 2022/30**

(21) Anmeldenummer: **18703558.9**

(22) Anmeldetag: **02.02.2018**

(51) Internationale Patentklassifikation (IPC):
***G06T 5/00*** *(2006.01)*  ***G06T 11/00*** *(2006.01)*

(52) Gemeinsame Patentklassifikation (CPC):
**G06T 11/005; G06T 5/001;** G06T 2207/10081; G06T 2207/30108

(86) Internationale Anmeldenummer:
**PCT/EP2018/052686**

(87) Internationale Veröffentlichungsnummer:
**WO 2018/141917 (09.08.2018 Gazette 2018/32)**

(54) **VERFAHREN ZUM DIMENSIONELLEN RÖNTGENOGRAPHISCHEN MESSEN, INSBESONDERE MITTELS COMPUTERTOMOGRAPHIE, UND RÖNTGEN-COMPUTERTOMOGRAPH**

METHOD FOR DIMENSIONAL X-RAY MEASUREMENT, IN PARTICULAR BY COMPUTED TOMOGRAPHY, AND X-RAY COMPUTED TOMOGRAPHY SCANNER

PROCÉDÉ DE MESURE DIMENSIONNELLE PAR RAYONS X, EN PARTICULIER PAR TOMODENSITOMÉTRIE ET TOMODENSITOMÈTRE À RAYONS X

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.02.2017 DE 102017102254**

(43) Veröffentlichungstag der Anmeldung:
**11.12.2019 Patentblatt 2019/50**

(73) Patentinhaber: **Bundesrepublik Deutschland, vertreten durch das Bundesmisterium für Wirtschaft und Energie, endvertreten durch den Präsidenten der PTB 38116 Braunschweig (DE)**

(72) Erfinder:
• **ILLEMANN, Jens**
 **38116 Braunschweig (DE)**
• **BARTSCHER, Markus**
 **31234 Edemissen (DE)**

(74) Vertreter: **Gramm, Lins & Partner Patent- und Rechtsanwälte PartGmbB Theodor-Heuss-Straße 1 38122 Braunschweig (DE)**

(56) Entgegenhaltungen:
**EP-A1- 2 644 095     US-A1- 2006 058 974**

• **FABRÍCIO BORGES DE OLIVEIRA ET AL: "Characterization and Correction of Geometric Errors Induced by Thermal Drift in CT Measurements", KEY ENGINEERING MATERIALS, Bd. 613, 21. Mai 2014 (2014-05-21), Seiten 327-334, XP055460076, DOI: 10.4028/www.scientific.net/KEM.613.327**

**Beschreibung**

Die Erfindung betrifft ein Verfahren zum dimensionellen Messen mittels Computertomographie gemäß dem Oberbegriff von Anspruch 1. Gemäß einem zweiten Aspekt betrifft die Erfindung einen Röntgen-Computertomographen gemäß dem Oberbegriff des unabhängigen Sachanspruchs.

Radiographie bezeichnet das Verfahren, bei dem ein Prüfling mit Röntgenstrahlung bestrahlt und die Intensität der Strahlung im Strahlungspfad hinter dem Prüfling als Bild gemessen wird. Computertomographie stellt eine Erweiterung zum Bestimmen eines dreidimensionalen Bildes dar, indem der Prüfling relativ zum Detektor und der Röntgenquelle gedreht und gegebenenfalls bewegt wird. Das dreidimensionale Volumenbild wird aus den Bildern in den verschiedenen Winkelstellungen, den Projektionen, mittels komplexer mathematischer Verfahren berechnet. In der industriellen Computertomographie werden bevorzugt Mikrofokusröntgenquellen und Flachbilddetektoren verwendet. Damit ist derzeit die relative Messunsicherheit bei der Bestimmung von Maßen auf $1 \times 10^{-4}$ beschränkt, wie internationale Vergleichsmessungen zeigen. Es ist wünschenswert, eine kleinere Messunsicherheit zu erreichen.

Nachteile bei der industriellen Computertomographie entstehen daraus, dass die verwendeten Röntgenquellen breitbandig Bremsstrahlung und charakteristische Strahlung emittieren, das heißt nicht-monochromatische Röntgenstrahlung. Zudem ist die maximale Leistung bei gegebenem Quellfleckdurchmesser beschränkt. Zwar wäre es möglich, beispielsweise eine Synchrotronstrahlungsquelle oder einen Freie-Elektronen-Laser zu verwenden, die diese Nachteile nicht haben, das aber erfordert eine dedizierte Großforschungseinrichtung, die für industrielle Messaufgaben im Routinebetrieb nicht genutzt werden kann.

Die Erfindung bezieht sich auf die Verwendung nicht-monochromatischer Röntgenstrahlung. Vorteilhafterweise werden mit Hilfe der Erfindung vergleichbar gute Resultate wie bei Verwendung monochromatischer Röntgenstrahlung ermöglicht. Als praktische Maßnahme wird bisher schon eine starke Vorfilterung der Röntgenstrahlung verwendet, um das Spektrum schmalbandiger zu machen auf Kosten einer geringeren Röntgenintensität. Das widerspricht dem, dass industrielle Computertomographie zudem in der Regel kurze Messzeiten erfordert, um wirtschaftlich zu sein. Das erfindungsgemäße Verfahren kann eine geringere Vorfilterung ermöglichen, damit die Messzeit reduzieren und ist damit wirtschaftlicher. Der Erfindung liegt weiterhin die Aufgabe zugrunde, die Messunsicherheit bei der Computertomographie, insbesondere der industriellen Computertomographie, zu verringern, die durch die Breitbandigkeit der Röntgenquelle systematisch negativ beeinträchtigt wird.

Aus dem Artikel Einfluss der Quellbewegung auf Reproduzierbarkeit und Antastabweichungen im Röntgen-Computertomografen von Weiss et al.: Proceedings Industrielle Computertomografie Tagung, Wels, Österreich, 2010, ist bekannt, dass die thermische Ausdehnung des Targets aufgrund des Beschusses mit den Elektronen zu einer Verlagerung des Brennflecks führen kann. In dem Artikel wird angegeben, dass eine Veränderung der Betriebsparameter wie Beschleunigungsspannung und Strahlstrom aufgrund der Erwärmung und der damit einhergehenden Bewegung des Quellorts zu Fehlern führen kann, wohingegen Strahlaufhärtung, Artefakte der Rekonstruktion über unbekannte Effekte im untersuchten Fall keine Rolle spielen. Auch bei vernachlässigbar kleinen Fluktuationen des Brennflecks kommt es bei diesem Verfahren zu systematischen Messunsicherheiten.

Aus dem Artikel Depth-of-interaction estimates in pixelated scintillator sensors using Monte Carlo techniques von Sharma et al. in Nuclear Instruments and Methods in Physics Research A 841 (2017), Seiten 117 - 123 wird beschrieben, wie die optischen Pfade von Photonen im Szintillationsdetektor von Parametern wie der Oberflächenrauigkeit und der Absorption an der Oberfläche und dem Festkörper abhängen.

Dabei wird davon ausgegangen, dass die gesamte Energie eines Photons beim ersten Streuevent abgegeben wird, auch eine Compton Streuung wird nicht modelliert. Der Effekt, dass bei schräg einlaufendem Strahl, abhängig von der spektralen Aufhärtung des Strahls, sich unterschiedliche nicht rotationssymmetrische Intensitäten auf benachbarten Pixeln ergeben, wird nicht beschrieben.

In der US 8,208,603 B2 ist beschrieben, wie sich der Anodenwinkel auf das Spektrum und die Intensitätsverteilung eines Nutzröntgenstrahlungskegels einer Röntgenröhre auswirkt und wie dieser Effekt korrigiert werden kann. Der Effekt der Strahlaufhärtung auf eine Veränderung der effektiven Eindringtiefe oder des effektiven Quellorts wird nicht beschrieben.

Der Erfindung liegt die Aufgabe zugrunde, die Messunsicherheit bei der Computertomographie (CT), insbesondere der industriellen Computertomographie, zu verringern.

Die Erfindung löst das Problem durch ein Verfahren mit den Merkmalen von Anspruch 1.

Gemäß einem zweiten Aspekt löst die Erfindung das Problem durch einen Röntgen-Computertomographen mit den Merkmalen des unabhängigen Sachanspruchs.

Der Erfindung liegt die Erkenntnis zugrunde, dass die vom Prüfling absorptionsbedingten Grauwerte benachbarter Pixel des Detektors bei radiografischen Aufnahmen, insbesondere in Kegelstrahlgeometrie, mit einem breiten Spektrum, anders als bei nahezu paralleler und/oder monochromatischer Röntgenstrahlung, aufgrund strahlungsphysikalischer Eigenarten der Quelle und des Detektors ein gerichtetes Übersprechen zeigen. Die dadurch entstehende lokale Änderung der Größenskalierung im radiografischen Bild überträgt sich auf ein rekonstruiertes CT-Bild, also ein dreidimensionales Dichtebild des Prüflings, das aus den pixelabhängigen Intensitätsdaten für verschiedene Orientierungen des Prüflings relativ zu Röntgenquelle und Detektor berechnet wird. Aus dem Dichtebild werden die Prüflingsoberflächen berechnet und Abmessungen bestimmt, die letztendlich durch das gerichtete Übersprechen im Grauwertbild verändert werden. Der lokale radiografische Vergrößerungsfaktor ergibt sich als der Quotient des Abstands vom wirksamen Quellpunkt zum wirksamen Detektionsort als Zähler zu dem Abstand zwischen dem wirksamen Quellpunkt und dem Prüfling als Nenner. Der Ort des Prüflings ist dabei bei der CT durch die Position der Rotationsachse gegeben, der effektive Quellpunkt und Detektionsort sind durch den Erwartungswert der Absorption bzw. Emission gemittelt über das im Prüfling absorbierte Spektrum auf dem Weg vom Quellpunkt zum Detektionsort gegeben.

Es ist bekannt, dass breitbandige Röntgenstrahlung beim Durchstrahlen des Prüflings eine Strahlaufhärtung zeigt. Hierunter ist das Phänomen zu verstehen, dass Röntgenstrahlung höherer Energie und damit kleinerer Wellenlänge normalerweise weniger stark absorbiert wird als Röntgenstrahlung geringer Energie und damit größerer Wellenlänge. Damit verändert sich das Röntgenspektrum mit einer relativen Zunahme des Anteils "harter", das heißt höherenergetischer Photonen.

Die Erfinder haben erkannt, dass die effektive Eindringtiefe der Röntgenstrahlung im Detektor umso größer ist, je stärker sie im Prüfling absorbiert wurde. Dieser Effekt ist unbeachtlich, solange der Röntgenstrahl senkrecht auf den Detektor auftrifft. Ist der Detektor daher so gekrümmt, dass ein Krümmungskreismittelpunkt mit dem Quellpunkt der Röntgenquelle übereinfällt, ergibt sich aus diesem Effekt kein Messfehler.

Gekrümmte flächige Detektoren sind jedoch schwierig herzustellen und werden daher in der Regel nicht eingesetzt. Das aber führt dazu, dass an solchen Stellen, an denen der Röntgenstrahl unter einem von 90° abweichenden Winkel auf den Detektor auftrifft, die Röntgenphotonen im Mittel umso weiter außen detektiert werden, je härter die Röntgenstrahlung ist.

Vorteilhafterweise kann die Erfindung mit der bereits bekannten Strahlaufhärtungskorrektur kombiniert werden, bei der Pixel für Pixel ihre Grauwerte mit einer nichtlinearen Funktion korrigiert werden, um die Abweichung der exponentiellen Schwächung der Intensität mit der Absorberdicke, bekannt als de Beer'sches Gesetz, zu berücksichtigen. Durch das Anbringen der erfindungsgemäßen Korrektur zusätzlich zur bekannten Strahlaufhärtungskorrektur, stimmt das radiographische Bild besser mit dem Bild überein, das ein idealer, das heißt sehr dünner, Detektor bei gegebenem Quellezu-Detektor-Abstand gemessen hätte - ein sehr dünner Detektor hat für Röntgenstrahlung aber keine hinreichende Absorption. Damit wird die Messunsicherheit reduziert.

Es ist ein weiterer Vorteil, dass diese Korrektur mathematisch einfach ist und damit schnell durchgeführt werden kann, ohne dass große Rechenkapazität bereitgestellt werden muss.

Ein weiterer Vorteil ist es, dass die Berechnung des dreidimensionalen Modells aus den korrigierten pixelabhängigen Intensitätsdaten auf die gleiche Weise durchgeführt werden kann, wie an nicht korrigierten pixelabhängigen Intensitätsdaten. In anderen Worten kann bestehende Software eingesetzt werden.

Vorteilhaft ist zudem, dass die Messzeit verringert werden kann, da, wie oben bereits dargestellt, auf eine starke Vorfilterung der Röntgenstrahlung verzichtet werden kann. Es kann nämlich wegen der Korrektur der effektiven Eindringtiefe auf dem Detektor und/oder einer Verschiebung des effektiven Quellorts Röntgenstrahlung verwendet werden, die eine größere Spektralbreite hat. In anderen Worten kann im Vergleich zu vorherigen Messstrategien mit einem schwächeren oder gar keinem Vorfilter gearbeitet werden. Das verkürzt die Messzeit.

Ein weiterer Vorteil ist es, dass größere Prüflinge vermessen werden können. Bei einer typischen Beschleunigungsspannung von U=225 Kilovolt können Prüflinge sinnvoll dimensionell vermessen werden, deren Materialstärke höchstens 75 Millimeter beträgt, wenn sie aus Aluminium gefertigt sind, oder höchstens 15 Millimeter beträgt, wenn sie aus Stahl bestehen. Diese Dicken entsprechen einer 95%igen Absorption, wenn die Strahlung mit 2 mm Kupfer vorgefiltert wurde. Es wurde empirisch festgestellt, dass die Messunsicherheit schon bei Absorption von mehr als 50% größer wird, ohne dass die Ursache für diesen Effekt zutreffend erkannt worden wäre. Da der Einfluss der Spektrumsabhängigkeit von Quell- und Detektorposition, die eine lokale Änderung des Schattenwurfs bewirken, nun bekannt und damit korrigierbar ist, können größere Absorptionen toleriert werden. In anderen Worten können Prüflinge mit größeren Abmessungen dimensionell gemessen werden.

Durch die erfindungsgemäße Korrektur ist eine relative Abstandsmessunsicherheit von unterhalb $3\times10^{-5}$ erreichbar. Insbesondere für leicht deformierbare Objekte, wie beispielsweise Kunststoffprodukte, liegt diese Messunsicherheit in dem Messbereich, der auch durch taktile Messungen erreicht wird. Es besteht daher die begründete Erwartung, dass Computertomographie auf Basis der Erfindung zukünftig taktile Messungen verstärkt ersetzen kann.

Zusätzlich zu dem oben beschriebenen Aspekt, dass die Strahlaufhärtung im Prüfling und Vorfilter die Lage des Schattenbildes im Detektormaterial beeinflusst, trägt ein weiterer daraus geometrisch zu verstehender Aspekt zur systematischen Messunsicherheit bei. Trifft ein Röntgenstrahl auf den Detektor, der stark geschwächt, also aufgehärtet, wurde, so haben dessen Photonen - wie oben erläutert - im Mittel eine höhere Energie. Das wiederum bedeutet, dass die Photonen mit erhöhter Wahrscheinlichkeit von einem Quellort auf dem Target stammen, der dicht an der Eintrittsstelle des Elektronenstrahls an Oberfläche liegt, weil die Elektronen dort noch eine besonders hohe kinetische Energie haben.

Entsprechend stammen die Photonen, die an einer Stelle auf den Detektor auftreffen, an dem eine hohe Intensität gemessen wird, mit einer relativ größeren Wahrscheinlichkeit von einer Stelle auf dem Target, an dem die Elektronen eine geringere Energie hatten. Das kann beispielsweise ein Punkt tiefer unterhalb der Oberfläche des Targets sein. Wird der Entstehungsort der Röntgenstrahlung als Punkt genähert, so hängt die Position dieses Punktes, und damit der Abstand zwischen diesem Punkt und dem Detektor einerseits sowie der Abstand zwischen diesem Punkt und dem Prüfling andererseits, von der im Detektor gemessenen Intensität ab. Diesen Einfluss auf das radiographische Bild zu korrigieren und damit die Messunsicherheit zu verringern, ist ein unabhängiger Gegenstand der Erfindung und zudem bevorzugt auch eine bevorzugte Ausprägung des oben beschriebenen Aspekts der Erfindung. In anderen Worten wird die Verschiebung des effektiven Quellorts korrigiert.

Im Rahmen der vorliegenden Beschreibung wird bei Verwendung eines unbestimmten Artikels stets auch gemeint, dass entweder genau eines oder mehrere der entsprechenden Objekte vorhanden sind. So kann genau eine Punktquelle vorhanden sein, aber auch zwei oder mehr Punktquellen.

Unter einer Punktquelle wird insbesondere eine Röntgenquelle verstanden, bei der der Quellort der Röntgenstrahlung in hinreichend guter Näherung als punktförmig angesehen werden kann. Hierunter ist insbesondere zu verstehen, dass diese Näherung zu einer systematischen Messunsicherheit von höchstens $10^{-5}$ führt. Insbesondere hat der Elektronenstrahl beim Auftreffen auf ein Target der Röntgenquelle einen Durchmesser (Durchmesser bei halbem Maximalwert) von höchstens 0,5 Millimeter, insbesondere höchstens 0,1 Millimeter. Vorzugsweise liegt die Röntgenstrahlung in Form eines Kegelstrahls oder eines Fächerstrahls vor.

Unter Computertomographie wird insbesondere auch eine Laminographie verstanden.

Unter einer Abmessung wird insbesondere auch ein geometrisches Maß oder ein geometrisches Merkmal verstanden.

Die Röntgenstrahlung ist nicht-monochromatisch. Vorzugsweise handelt es sich um Bremsstrahlung mit einem Anteil charakteristischer Strahlung des Targetmaterials. Günstig ist es, wenn die Röntgenstrahlung durch Bestrahlung eines Targets mit Elektronen hergestellt wird, wobei eine Energie der Elektronen vorzugsweise zwischen 20 und 600 Kiloelektronenvolt, vorzugsweise zwischen 60 und 225 Kiloelektronenvolt, liegt.

Unter einem Bewegen des Prüflings relativ zur Röntgenquelle wird ein Bewegen des bei Nichtbewegen der Röntgenquelle, ein Bewegen der Röntgenquelle bei Nichtbewegen des Prüflings oder ein Bewegen beider verstanden.

Unter dem Merkmal, dass die pixelabhängigen Intensitätsdaten um den Einfluss einer strahlaufhärtungsbedingten Veränderung der effektiven Eindringtiefe korrigiert wird, wird verstanden, dass die Intensitätsdaten so verändert werden, dass der Effekt korrigiert wird, dass die von einem Pixel gemessene Intensität positiv mit der effektiven Eindringtiefe korreliert, dass also eine hohe Intensität auf eine hohe effektiven Eindringtiefe schließen lässt.

Unter dem Merkmal, dass die Verschiebung des effektiven Quellorts auf einem Target der Röntgenquelle korrigiert wird, wird insbesondere verstanden, dass die strahlaufhärtungsbedingte Verschiebung des effektiven Quellorts korrigiert wird.

Vorzugsweise ist der Detektor ein Szintillationsdetektor oder ein volumenabsorbierender Halbleiterdetektor. Günstig ist es, wenn dieser Mikrosäulen aus szintillierendem Material aufweist. Das szintillierende Material ist beispielsweise mit Thallium dotiertes Cäsiumjodid oder enthält eine Gadolinium-, Wolfram- und/oder eine Lanthanverbindung.

Vorzugsweise beträgt eine Dicke einer Detektorschicht, insbesondere die Höhe der Mikrosäulen, zumindest 400 Mikrometer, insbesondere zumindest 500 Mikrometer. In diesem Fall ist der Einfluss der Spektrumsabhängigkeit der effektiven Eindringtiefe des Schattenwurfbildes des Prüflings besonders groß.

Die Verschiebung des Quellortes ist in der Regel jedoch nicht nur strahlaufhärtungsbedingt. Eine Verschiebung des Quellorts ergibt sich zudem aus der Kombination des Einflusses der Strahlaufhärtung und der spektral veränderlichen Röntgen-Emission über den Weg des strahlungserzeugenden Elektronenstrahls im Röntgentarget. Vorzugsweise wird dieser Einfluss ebenfalls korrigiert

Durch die Schritte: für zumindest eine Mehrzahl der Pixel, insbesondere alle Pixel, (i) Ermitteln eines Nullpunkt-Abstands des Pixels von einer optischen Achse, (ii) Ermitteln der Intensität der von dem Pixel gemessenen Röntgenstrahlung, (iii) Zuweisen einer korrigierten Position, die vom Nullpunkt-Abstand und der Intensität abhängt, und (iv) aus allen korrigierten Positionen und den zugehörigen Intensitäten Berechnen von korrigierten pixelabhängigen Intensitätsdaten, kann aus diesen korrigierten pixelabhängigen Intensitätsdaten ein dreidimensionales Modell des Prüflings berechnet werden. Diese Berechnung unterscheidet sich insbesondere nicht von einem Verfahren, das aus dem Stand der Technik bekannt ist.

Es sei darauf hingewiesen, dass Ermitteln der Intensität der von dem Pixel gemessenen Röntgenstrahlung auch ein Ermitteln der Intensität der von dem Pixel gemessenen absorbierten Röntgenstrahlung umfasst.

Unter der optischen Achse wird insbesondere diejenige Gerade verstanden, entlang der ein gedachter Röntgenstrahl von der Röntgenquelle zum Detektor verläuft, wobei dieser Röntgenstrahl senkrecht zu einer Detektorfläche verläuft, entlang der sich der Detektor erstreckt.

Die absoprtionsspektrumsbedingte effektive Quell- und Detektorortverschiebung hat keinen Einfluss auf die detektierte Position eines gedachten Röntgenstrahls zu einem Detektorpixel, sofern das betreffende Pixel auf der optischen Achse liegt. Je größer der Abstand eines Pixels von der optischen Achse ist, desto größer wird die durch die Verschiebungen bedingte Messortabweichung.

Die beiden Korrekturen, nämlich des Quellortes und der Eindringtiefe im Detektor, können in verschiedenen Bereichen der Intensitätsdaten unterschiedlich sein.

Unter dem Ermitteln der Intensität wird insbesondere verstanden, dass ein Messwert aufgenommen wird, der die Intensität der Röntgenstrahlung beschreibt. Beispielsweise handelt es sich bei der so ermittelten Intensität um einen Graustufenwert, der die Intensität kodiert, die von dem entsprechenden Pixel gemessen wird.

Vorzugsweise wird die Korrektur der pixelabhängigen Intensitätsdaten so durchgeführt, dass die korrigierte Position, der Nullpunkt und die ursprüngliche Position des entsprechenden Pixels auf einer Linie liegen. Der Nullpunkt ist der Punkt auf dem Detektor, der den Abstand Null von der optischen Achse hat. Wird in anderen Worten der Detektor in einem Polarkoordinatensystem betrachtet, so ändert die Korrektur um den Einfluss der Eindringtiefe zwar die Abstandskoordinate, nicht aber den Azimutalwinkel.

Um den Effekt der effektiven Quell- und/oder Detektorortverschiebung für jedes Pixel zu kompensieren, wird die Auswirkung des Effektes mit negativen Vorzeichen so auf das Bild durchgeführt, dass ein Differenz-Abstand $\Delta r$ zwischen dem Nullpunkt-Abstand der korrigierten Position und dem Nullpunkt-Abstand der unkorrigierten Position r im Wesentlichen berechnet wird aus einem Produkt aus dem Abstand r, der absorbierten Intensität $(I_0-I)$, die von dem jeweiligen Pixel gemessen wird, und einem Intensitätskorrekturparameter k und einer von Filter- und Prüflingsmaterial abhängigen Konstante c: $\Delta r = r * ((I_0-I) * k + c)$.

Eine zum Abstand r proportionale radiale Verschiebung der Pixel beschreibt eine Veränderung des geometrischen Vergrößerungsfaktors eines industriellen CT mit Punktquelle und Flachbilddetektor. Die Konstante c beschreibt eine Veränderung der Vergrößerung für das ganze Bild durch die Lage des Absorptionsbildes einer dünnen Schicht des Prüflingsmaterials im Detektor. Dies hängt von dem Spektrum der Röntgenquelle, also insbesondere Beschleunigungsspannung, Targetmaterial, Filtermaterial und -dicke ab. Dieser Intensitätskorrekturparameter k ist vom Prüflingsmaterial, den Quellbedingungen, insbesondere Beschleunigungsspannung und Targetmaterial, und dem Detektor, insbesondere Material und Dicke, abhängig.

Der Intensitätskorrekturparameter ist hingegen, zumindest in linearer Ordnung des Nullpunkt Abstands, nicht vom Nullpunkt-Abstand r selbst abhängig. Insbesondere ist der Intensitätskorrekturparameter in guter Näherung nicht von der Intensität abhängig, wie experimentelle Ergebnisse nahelegen. Er beschreibt empirisch, dass mit Veränderung der absorbierten Intensität eine Strahlaufhärtung eintritt und damit eine veränderte Eindringtiefe in das Detektormaterial in der Umgebung des entsprechenden Pixels. Entsprechendes gilt auch für die Quellortverschiebung. Dies führt lokal an dieser Stelle zu einer veränderten Vergrößerung und damit zu einer Verschiebung der Intensität von diesem Pixel auf benachbarte. Die Parameter k und c können für verschiedene Bereiche des Bildes unterschiedlich angenommen werden, wenn unterschiedliche Prüflingsmaterialien in diesen zur Absorption beitragen.

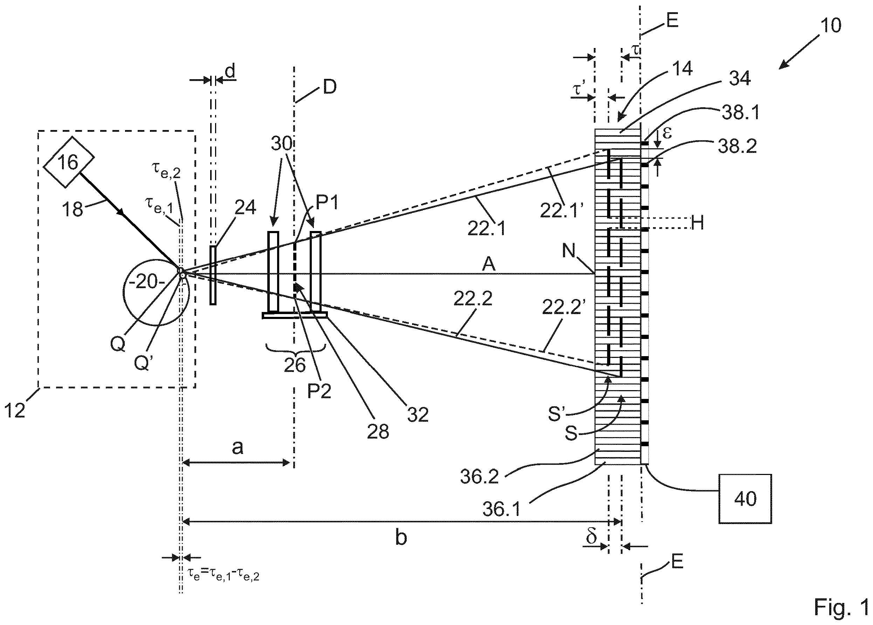

Die Formel oben gilt für den radialsymmetrischen Fall, der hervorgerufen wird durch die Detektorortverschiebung $\delta$. Bei der Quellortverschiebung, insofern der Quellort gerichtet entlang eines geneigten Targets mit einer Komponente parallel zum Detektor wandert, ist diese Symmetrie gebrochen. Es kann dieses dadurch berücksichtigt werden, indem pixelweise eine zusätzliche Verschiebung in Y-Richtung (in Richtung Targetneigung wie in Fig. 1) angebracht wird. Diese ist allerdings näherungsweise unabhängig von der Y-Position auf dem Detektor, aber proportional zur absorbierten Intensität: $\Delta r = (I_0-I) * w$, wobei w eine Konstante mit ähnlichen Abhängigkeiten wie k ist.

Vorzugsweise wird das Korrigieren des Einflusses der Eindringtiefe so durchgeführt, dass ein Differenz-Abstand zwischen dem Nullpunkt-Abstand der korrigierten Position und dem Nullpunkt-Abstand der unkorrigierten Position im Wesentlichen berechnet wird aus einem Produkt aus der Intensität, die von dem jeweiligen Pixel gemessen wird, und einem Intensitätskorrekturparameter. Dieser Intensitätskorrekturparameter ist vorzugsweise eine Konstante, insbesondere ist der Intensitätskorrekturparameter, zumindest in linearer Ordnung des Nullpunkt-Abstands, nicht vom Nullpunkt-Abstand abhängig. Insbesondere ist der Intensitätskorrekturparameter zudem nicht von der Intensität abhängig.

Unter dem Merkmal, dass der Differenz-Abstand im Wesentlichen wie angegeben berechnet wird, wird insbesondere verstanden, dass es zwar möglich, nicht aber notwendig ist, dass in die Berechnung des Differenz-Abstands weitere Parameter eingehen. Vorzugsweise ist der etwaige zusätzliche Beitrag, der nicht das Produkt aus Intensität und Intensitätskorrekturparameter ist, kleiner als ein Drittel, insbesondere kleiner als ein Fünftel des Produkts aus Intensität und Intensitätskorrekturparameter.

Vorzugsweise wird die Röntgenstrahlung durch Bestrahlen eines Quellpunkt eines Targets mit Elektronen erzeugt. Das Berechnen der Abmessung des Prüflings erfolgt vorzugsweise anhand eines Vergrößerungsfaktors, der sich aus dem Quotienten des Abstands b des Quellpunkts vom Detektor und einem Abstand a des Prüflings vom Quellpunkt berechnet. Der Vergrößerungsfaktor wird vorzugsweise um den Einfluss einer Elektronen-Eindringtiefe in das Target korrigiert. Dies erfolgt beispielsweise ebenfalls dadurch, dass wie oben beschrieben der Differenzabstand entsprechend berechnet wird. In anderen Worten kann durch die oben angegebene Berechnung sowohl der Einfluss der Eindringtiefe im Detektor als auch der Einfluss einer Verschiebung des effektiven Quellpunkts aufgrund von Strahlaufhärtung kompensiert werden.

Der Vergrößerungsfaktor wird vorzugsweise dadurch bestimmt, dass ein, bevorzugt dünner, Kalibrierkörper, dessen Abmessungen bekannt sind, gemessen wird. Das erfolgt gemäß der Schritte (a) bis (c) gemäß Anspruch 1. Nachfolgend wird die Dicke des (Vor-)filters verändert, mit dem der Röntgenstrahl gefiltert wird, bevor er auf den Kalibrierkörper trifft, und wiederholt gemessen. Der Intensitätskorrekturparameter wird dann aus einer Änderung der Vergrößerung des Schattenbilds des Kalibrierkörpers auf den Detektor in Abhängigkeit von der Intensität der Röntgenstrahlung berechnet. Durch das Verändern der Filterstärke des Filters ändert sich die Intensität, die bei einem gegebenen Pixel gemessen wird, sowie auch das Röntgenspektrum.

Die genauen Quellort- und Detektorortverschiebungen können folgendermaßen bestimmt werden, woraus c und v berechnet werden können: es müssen mindestens vier Messungen bei verschiedenen gemessenen geometrischen Vergrößerungen V1 bis V4 mit unterschiedlichen Positionen des Kalibrierkörpers unter ansonsten gleichen Bedingungen durchgeführt werden, so dass man die Quellort ($\tau_e$)- und Detektorortverschiebungen ($\delta$) separieren kann. Es ergibt sich ein Gleichungssystem:

$$V1 = (b + \delta + \tau_e) / (a + \tau_e)$$

$$V2 = (b + d1 + \delta + \tau_e) / (a + d1 + \tau_e)$$

$$V3 = (b + d2 + \delta + \tau_e) / (a + d2 + \tau_e)$$

$$V4 = (b + d3 + \delta + \tau_e) / (a + d3 + \tau_e)$$

Messungen in mehr verschiedenen Positionen sind günstig, da sich durch die Überbestimmung des Gleichungssystems statistisch genauere Ergebnisse berechnen lassen. d1 bis d3 stellen Verschiebungen des Prüfkörpers zur Ausgangslage dar, die bevorzugt mittels eines Laserinterferometers bestimmt werden können.

Die experimentell bestimmten Vergrößerungen V1 bis V4 ergeben sich als Quotient aus der Größe des Bilds auf dem Detektor 14 und den bekannten Abmessungen des Kalibrierkörpers. Die Größe des Bilds auf dem Detektor ergibt sich aus den Abmessungen des Bildes in Pixeln multipliziert mit dem bekannten Abstand zweier Pixel. Um den Versatz des Kalibrierkörpers von der Drehachse zu berücksichtigen, können die Messungen in 0°- und 180°-Stellung der Achse wiederholt und ein Mittelwert gebildet werden. Bevorzugt kann als Kalibrierkörper eine gleichmäßige Gitterstruktur verwendet werden, deren Gitterkonstante als Maßverkörperung verwendet wird.

Damit sind a, b, $\delta$ und $\tau_e$ unbekannte Variable, die aus dem Gleichungssystem mit vier Gleichungen bestimmt werden können. Mit den nun bekannten Parametern lässt sich der für die Bildkorrektur nötige Parameter c bei gegebener Vergrößerung berechnen. Typische Werte von c für die bereits oben beschriebenen bevorzugten Parameter des Tomographen sind 100 $\mu$m bis 300 $\mu$m, womit sich die Vergrößerung, bei zum Beispiel einem Meter Abstand von Detektor zu Quelle, um relativ $1 \cdot 10^{-4}$ bis $3 \cdot 10^{-4}$ verändert. Durch Verwendung verschiedener, bevorzugt dünner, Kalibrierkörper aus verschiedenem Material und unter verschiedenen Bedingungen der Strahlquelle können relative Änderungen von c bestimmt werden, indem die geometrischen Vergrößerungsfaktoren bei ansonsten gleichen geometrischen Verhältnissen verglichen werden. Dies erspart die Messungen in verschiedenen Positionen, um c für andere Betriebsbedingungen und Prüflingsmaterialien zu bestimmen.

Günstig ist es, wenn bei dem Vermessen eines Prüflings, der kein Kalibrierkörper ist, ein Filter verwendet wird, das höchstens 75 % der Gesamt-Intensität der Rohstrahlung herausfiltert. Auf diese Weise ergibt sich eine besonders geringe Messzeit. Dieses bedingt gleichzeitig ein breites Röntgenspektrum, das ohne die erfindungsgemäße Korrektur der radiographischen Bilder zu besonders hohen Abweichungen führt. Andersherum bedeutet dies, dass man unter Hinzuziehung des erfindungsgemäßen Verfahrens mit geringer Filterung und damit kürzerer Messzeit arbeiten kann, was einen wirtschaftlichen Vorteil darstellt.

Im Folgenden wird die Erfindung anhand der beigefügten Zeichnungen näher erläutert. Dabei zeigt

Figur 1    schematisch einen erfindungsgemäßen Röntgen-Computertomographen zum Durchführen eines erfindungsgemäßen Verfahrens,

Figur 2    mit den Teilfiguren 2a und 2b zeigt schematisch, wie die pixelabhängigen Intensitätsdaten um den Einfluss der Eindringtiefe auf den Faktor korrigiert werden und

Figur 3    mit den Teilfiguren 3a und 3b zeigt experimentelle Daten, in denen die Abhängigkeit der Vergrößerung von der absorbierten Intensität gezeigt wird.

Figur 1 zeigt eine schematische Ansicht eines erfindungsgemäßen Röntgen-Computertomographen 10, der eine Röntgenquelle 12 und einen Detektor 14 aufweist. Die Röntgenquelle 12 besitzt eine Elektronenstrahlquelle 16 zum Erzeugen eines Elektronenstrahls 18, der auf ein Target 20 gerichtet ist. Das Target 20 besteht beispielsweise aus Wolfram. Die Elektronen des Elektronenstrahls 18 haben eine Energie von beispielsweise 225 Kiloelektronenvolt. Der Elektronenstrahl 18 trifft in einem Quellpunkt Q auf das Target 20. Ein Auftreffwinkel zwischen dem Elektronenstrahl 18 und einer Oberfläche des Targets 20 liegt vorzugsweise zwischen 15 und 30 °. Alternativ kann ein dünnes Target auch rückseitig durchstrahlt werden.

Beim Auftreffen der Elektronen des Elektronenstrahls 18 auf das Target 20 entsteht Röntgenstrahlung, die zur vereinfachten Betrachtung als aus mehreren Röntgenstrahlen 22.i zusammengesetzt betrachtet werden kann. In der Zeichnung sind zwei Strahlengänge mit Index i = 1, 2 eingezeichnet. In Strahlrichtung hinter dem Target 20 ist ein optionales Filter 24 angeordnet, das eine Strahlaufhärtung der Röntgenstrahlen 22.i bewirkt. Das Filter besteht beispielsweise aus Aluminium oder Kupfer und hat eine Dicke d.

Im Strahlungspfad hinter dem Filter 24 ist ein Prüfling 26 angeordnet. Der Prüfling 26 beinhaltet eine zu messende Struktur 28, beispielsweise eine Bohrung, und die Struktur 28 umgebenes Material 30. Diese gedachte Unterteilung des Prüflings 26 in Struktur 28 und Material 30 dient nur der Erläuterung der Erfindung und soll keine einschränkende Aussage über die Art des Prüflings enthalten.

Der Röntgen-Computertomograph 10 umfasst vorzugsweise eine Probenaufnahme zum Aufnehmen des Prüflings 26. Die Probenaufnahme ist vorzugsweise als Bewegungsvorrichtung 32, insbesondere als Drehvorrichtung zum Drehen des Prüflings 26 um eine Drehachse D, ausgebildet. Die Drehachse D hat einen ersten Abstand a vom Quellpunkt Q.

Der Detektor 14 ist in Strahlrichtung hinter dem Prüfling 26 angeordnet und hat einen zweiten Abstand b vom Quellpunkt Q. Der Detektor 14 weist im vorliegenden Fall ein Szintillationselement 34 auf, das eine Vielzahl an Mikrosäulen 36 aufweist. Die Mikrosäulen erstrecken sich senkrecht zu einer Detektorebene E und bestehen beispielweise aus Cäsiumjodidkristallitnadeln. Trifft ein Röntgenquant auf den Detektor 14, so entsteht ein Lichtblitz, der sich entlang der benachbarten Mikrosäulen ausbreitet und so auf eine kleine Anzahl an Photoelementen 38i trifft. Es sei darauf hingewiesen, dass der Laufindex i für mehrere Objekte verwendet wird, ohne dass eine Zuordnung damit gemeint ist.

Figur 1 zeigt zwei Szenarien für ein Schattenbild S, S' des Prüflings 26 auf dem Detektor 14. Für das erste Szenario sind gestrichelt zwei Röntgenstrahlen 22.1', 22.2' ausgehend vom Quellpunkt Q' eingezeichnet, die dem Fall entsprechen, dass kein Filter 24 vorhanden ist und der Prüfling 26 lediglich aus der Struktur 28 aufgebaut ist. Als Folge kommt es nur zu minimaler Strahlaufhärtung und einer hohen Intensität, die vom Detektor 14 gemessen wird. Aufgrund der geringen Strahlaufhärtung ist die effektive Eindringtiefe $\tau'$ vergleichsweise klein. Mit durchgezogener Linie sind für das zweite Szenario zwei Röntgenstrahlen bezeichnet, die durch die gleichen Punktpaare P1 und P2 der Struktur 28 laufen, wobei ein Filter 24 vorhanden ist und/oder die Struktur 28 von einer signifikanten Menge an Material 30 umgeben ist. Wie in der Beschreibungseinleitung dargelegt, kommt es zu einer Strahlaufhärtung und damit zu einer größeren effektiven Eindringtiefe $\tau$ in den Detektor 14. Die Differenz $\delta = \tau - \tau'$ ist größer als Null. Die Wegstrecke von der Quelle zum Detektor b wird daher um diesen Wert größer und das Abbild der Punkte P1 und P2 liegt im Verhältnis $(b + \delta)/b$ weiter auseinander als im Originalbild. Als zweite Auswirkung der zusätzlichen Strahlaufhärtung durch den Absorber 30 ist der mittlere Quellort Q auf dem Target ein anderer. Da das Target schräg zur Achse A verläuft, vergrößern sich gleichzeitig die Abstände a und b um den Wert $\tau_e$, wie auch ein seitlicher Versatz auftritt, der geometrisch vergrößert als Versatz $\varepsilon$ im Bild in Richtung der Targetneigung auftritt. Die Messergebnisse des Detektors 14 werden mittels einer Auswerteeinheit 40 ausgewertet, die dazu mindestens einen Prozessor und einen digitalen Speicher besitzt.

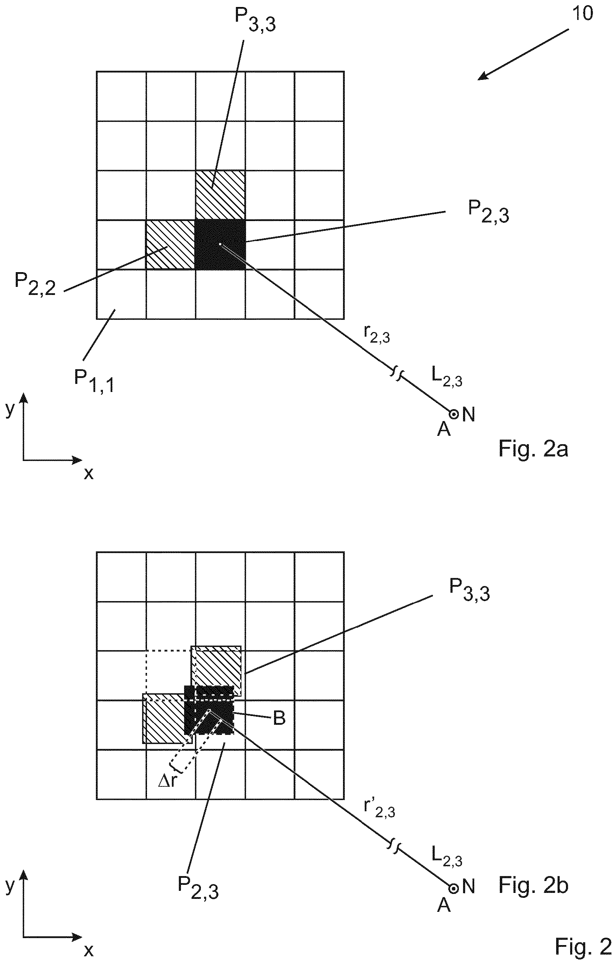

Figur 2a zeigt, wie der beschriebene Effekt korrigiert werden kann und stellt dazu schematisch einen Ausschnitt aus dem Detektor 14 mit den Pixeln $P_{x,y}$ dar. Das Pixel $P_{2,3}$ detektiert eine sehr geringe Intensität $I_{2,3} = I(P_{2,3})$. Hingegen detektieren die Pixel $P_{3,3}$ und $P_{2,2}$ eine mittlere Intensität $I_{3,3}$ bzw. $I_{2,2}$. Für die übrigen Pixel P wird angenommen, dass sie der Einfachheit halber eine maximale Intensität $I_{x,y} = I_{max}$ detektieren.

Das Pixel $P_{2,3}$ hat einen Nullpunkt-Abstand $r_{2,3}$ von einem Nullpunkt N (siehe Figur 1) des Detektors 14 auf der optischen Achse A (vgl. Figur 1). Die optische Achse A ist diejenige Linie, die durch den Quellpunkt Q verläuft und senkrecht auf der Detektorebene E steht, entlang der sich der Detektor 14 erstreckt. Es wird näherungsweise der Quellpunkt verwendet, der detektiert wird, wenn weder ein Filter noch ein Prüfling im Aufbau vorhanden sind.

Figur 2b zeigt, dass die pixelabhängigen Intensitätsdaten dadurch um den Einfluss der Eindringtiefe $\tau$ und der Quellortverschiebung $\tau_e$ korrigiert werden, dass der Intensität $I(P_{2,3})$, also der Intensität, die von dem Pixel $P_{2,3}$ gemessen wird, eine neue Position K zugewiesen wird. Diese neue Position K wird berechnet durch Verschieben der Position des ursprünglichen Pixels $P_{2,3}$ in Richtung der Verbindungslinie $L_{2,3}$ von der optischen Achse A zur ursprünglichen Position des Pixel $P_{2,3}$. Der neue Abstand $r'_{2,3}$ berechnet sich zu $r'_{2,3} = r_{2,3}(1 + k \cdot I_{2,3} + c)$ mit dem Verschiebeparameter k, der materialabhängigen Konstante c und der Intensität I. Der Einfachheit halber wird hier c = 0 angenommen.

Wie Figur 2b schematisch zeigt, entspricht dies einer gedachten Verschiebung der Position des Pixels $P_{2,3}$ relativ zum ursprünglichen Pixelmuster und damit dem ursprünglichen Koordinatensystem. Auf die gleiche Weise wird die entsprechende Verschiebung für alle Pixel $P_{x,y}$ berechnet. Das ist für die Pixel $P_{3,3}$ und $P_{2,2}$ angedeutet.

In einem nachfolgenden Schritt wird jedem Pixel $P_{x,y}$ eine korrigierte Intensität $I'_{x,y}$ zugewiesen. Das erfolgt dadurch, dass für jedes Pixel berechnet wird, welchen Flächenanteil die berechnete verschobene Intensität am jeweiligen Pixel hat. So wird dem Pixel $P_{2,3}$ die Intensität $I'_{2,3}$ zugewiesen, die im vorliegenden Fall dem 0,52-fachen der Intensität $I_{2,3}$ entspricht, da lediglich 52 % der schwarzen Fläche in dem Bereich des Pixels $P_{2,3}$ liegen, wie Figur 2b zu entnehmen ist. Dieser Bereich B ist in Figur 2b strichpunktiert umrandet. Die Intensität $I'_{3,3}$, die dem Pixel $P_{3,3}$ zugewiesen wird, ist $I'_{3,3}=I_{2,3}*0,22+0,82*I_{33}$. Diese Berechnung wird für alle Pixel $P_{x,y}$ des Ursprungsbilds des Detektors 14 durchgeführt. Die so korrigierten Intensitätsdaten ergeben ein korrigiertes Bild des Detektors 14 und werden danach zur Rekonstruktion eines dreidimensionalen Dichtebilds des Prüflings 26 verwendet.

Es sei darauf hingewiesen, dass es günstig, nicht aber notwendig ist, dass die Intensitäten für das ursprüngliche Pixelmuster berechnet werden. Es ist durchaus auch denkbar und von der Erfindung umfasst, dass diese pixelweise Intensitätskorrektur, die Intensität auf andere Nachbarpixel umverteilt, auf ein anderes Pixelmuster angewendet wird, beispielsweise ein hexagonales Gitter.

Figur 1 zeigt, dass sich aus dem ersten Abstand a und dem zweiten Abstand b ein Vergrößerungsfaktor $V = b / a$ berechnen lässt, der angibt, wie vielfach größer das Schattenbild S, S' gegenüber der Struktur 28 auf dem Detektor 14 vergrößert erscheint. Um eine Abmessung, beispielsweise ein Höhe H einer Ausnehmung im Prüfling 26 zu messen, muss dieser Vergrößerungsfaktor V bekannt sein.

Die Vergrößerungsfaktoren V1 bis V4 sowie der Abstände a und b werden oben erläutert. Die Vergrößerung ist eine unmittelbare Messgröße, wenn man die Abmessungen des Kalibriergitters kennt und die Pixelabstände des Detektors als bekannt (z.B. 200 $\mu$m) voraussetzt. Diese sind erstens sehr gut bekannt und zweitens ergibt es sich, dass die Detektorpixelgröße bei den Ergebnissen herausfällt, da alle Abmessungen in Pixel/Voxel ausgemessen werden und mit dem Kalibriergegenstand ins Verhältnis gesetzt werden.

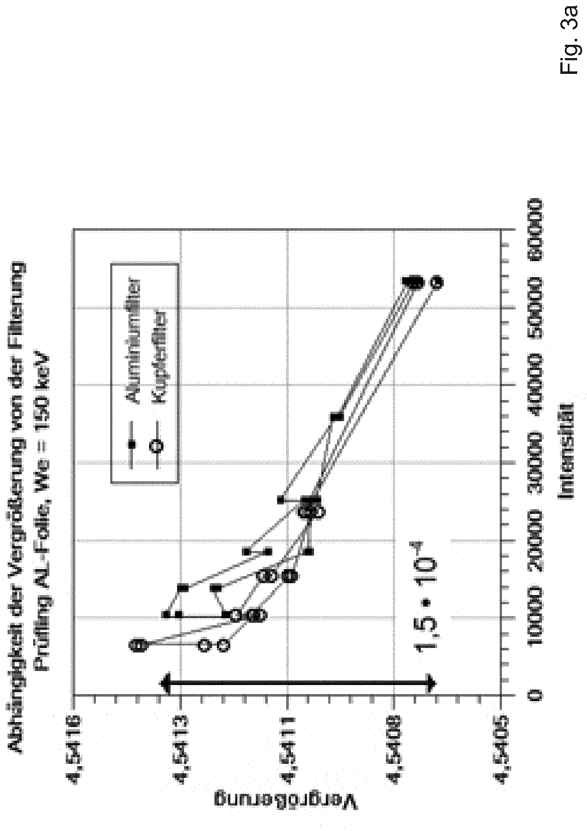

Figur 3a zeigt als x-Achse die vom Detektor gemessene Intensität I. Die Intensität wird durch zunehmend dickere Vorfilter entweder aus Kupfer (Kreise) oder aus Aluminium (Quadrate) verändert. Der Prüfling 26 besteht nur aus einer Struktur 28 in Form einer Aluminiumfolie mit mehreren Ausnehmungen, die an bekannten Positionen angeordnet sind. Die y-Achse gibt den Vergrößerungsfaktor V an. Es ist zu erkennen, dass der Vergrößerungsfaktor V mit zunehmender Intensität I abnimmt bzw. mit stärkerer Absorption zunimmt. Der Grund dafür ist der oben beschriebene Einfluss der zunehmenden Strahlaufhärtung auf die Eindringtiefe und die scheinbare Position des Quellpunkts.

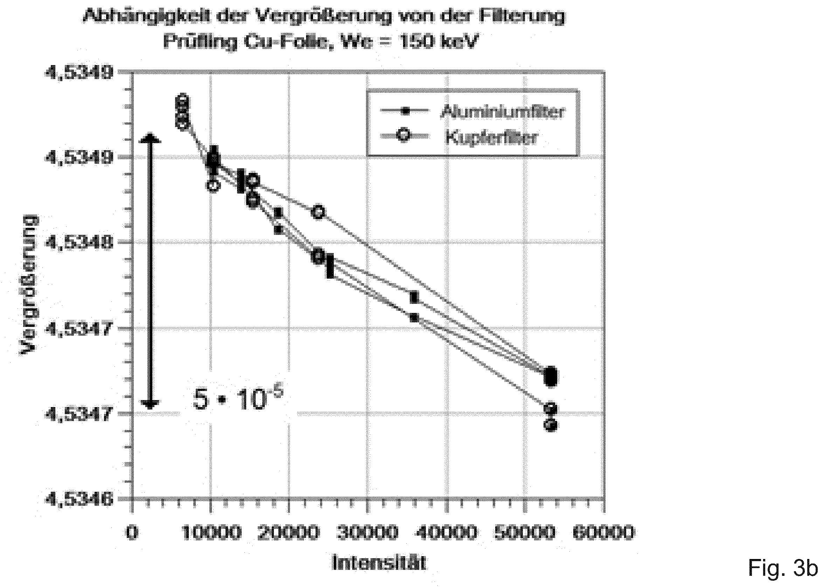

Figur 3b zeigt ein Diagramm wie in Figur 3a, wobei der Prüfling 26 nur aus einer Struktur 28 in Form einer Kupferfolie mit mehreren Ausnehmungen, die an bekannten Positionen angeordnet sind, besteht.

Bezugszeichenliste

| | | | |
|---|---|---|---|
| 10 | Röntgen-Computertomograph | P | Pixel |
| 12 | Röntgenquelle | Q | Quellpunkt |
| 14 | Detektor | r | Abstand |
| 16 | Elektronenstrahlquelle | S | Schattenbild |
| 18 | Elektronenstrahl | k | Intensitätskorrekturparameter |
| 20 | Target | V | Vergrößerungsfaktor |
| 22 | Röntgenstrahl | w | Konstante |
| 24 | Filter | | |
| 26 | Prüfling | $\delta$ | Differenz |
| 28 | Struktur | $\varepsilon$ | Verschiebung |
| | | $\tau$ | effektive Eindringtiefe |
| 30 | Material | $\tau e$ | effektive Quellpunktverschiebung |
| 32 | Probenaufnahme, Drehvorrichtung | | |
| 34 | Szintillationselement | | |
| 36 | Mikrosäule | | |
| 38 | Photoelement | | |
| 40 | Auswerteeinheit | | |
| a | erster Abstand | | |
| A | optische Achse | | |
| b | zweiter Abstand | | |
| d | Filterstärke | | |
| D | Drehachse | | |
| E | Detektorebene | | |
| H | Höhe | | |

(fortgesetzt)

| I | Intensität |
| K | Position |
| L | Abstand zur Bildmitte |

**Patentansprüche**

1. Verfahren zum dimensionellen röntgenographischen Messen, insbesondere mittels Computertomographie, mit den Schritten:

   a) Bestrahlen eines Prüflings (26) mit nicht-monochromatischer Röntgenstrahlung einer quasi-punktförmigen Röntgenquelle (12),
   b) Messen von Intensität (I) der Röntgenstrahlung (22) im Strahlungspfad hinter dem Prüfling (26) mittels eines Flachbilddetektors (14), der eine Vielzahl an Pixeln (P) aufweist, sodass pixelabhängige Intensitätsdaten (I(P)) erhalten werden, und
   c) Berechnen zumindest einer Abmessung (H) des Prüflings (26) anhand der pixelabhängigen Intensitätsdaten (I(P)), **dadurch gekennzeichnet, dass**
   d) die pixelabhängigen Intensitätsdaten (I(P)) um den Einfluss

   - einer strahlaufhärtungsbedingten Veränderung einer effektiven Eindringtiefe ($\tau$) in den Flachbilddetektor (14) und/oder
   - einer strahlaufhärtungsbedingten Verschiebung des effektiven Quellorts (Q) auf einem Target (20) der Röntgenquelle (12) korrigiert werden und das Korrigieren die folgenden Schritte umfasst:
   für zumindest eine Mehrzahl der Pixel (P)

   i) Ermitteln eines Nullpunkt-Abstands (r) des Pixels (P) von einer optischen Achse (A),
   ii) Ermitteln der Intensität (I) der von dem Pixel gemessenen Röntgenstrahlung,
   iii) Zuweisen einer korrigierten Position (K'), die vom Nullpunkt-Abstand (r) und der Intensität (I) abhängt, und
   iv) aus allen korrigierten Positionen (K') und den zugehörigen Intensitäten (I') Berechnen von korrigierten pixelabhängigen Intensitätsdaten (I'(P)).

2. Verfahren nach Anspruch 1, **dadurch gekennzeichnet, dass** die korrigierte Position (K'), der Nullpunkt (N) und die ursprüngliche Position (K) auf einer Linie liegen.

3. Verfahren nach Anspruch 1 oder 2, **dadurch gekennzeichnet, dass** ein Differenz-Abstand ($\Delta r$) zwischen dem Nullpunkt-Abstand (r') der korrigierten Position (K') und dem Nullpunkt-Abstand (r) der unkorrigierten Position (K) berechnet wird aus einem Term (c+k f(I)), der ein Produkt (k f(I)) aus einer Funktion (f) der Intensität (I), einem Intensitätskorrekturparameter (k) und einer Konstanten (c) enthält.

4. Verfahren nach einem der vorstehenden Ansprüche, **dadurch gekennzeichnet, dass**

   die Röntgenstrahlung durch Bestrahlen eines Quellpunkts (Q) eines Targets (20) mit Elektronen erzeugt wird, das Berechnen der Abmessung (H) des Prüflings (26) anhand eines Vergrößerungsfaktors ($\alpha$) erfolgt, der

   von einem Abstand (b) des Quellpunkts (Q) vom Flachbilddetektor (14) und
   einem Abstand (a) des Quellpunkts (Q) vom Prüfling (26) abhängt, und dass

   der Vergrößerungsfaktor ($\alpha$) um den Einfluss eines sich aufgrund einer sich ändernden Elektronen-Eindringtiefe in das Target (20) ändernden Röntgenemissionsspektrums korrigiert wird.

5. Verfahren nach Anspruch 3, **gekennzeichnet durch** die Schritte:

   - Vermessen eines Prüflings (26) in Form eines Kalibrierkörpers,
   - Verändern einer Filterstärke (d) eines Filters (24), mit dem die Röntgenstrahlung gefiltert wird, bevor sie auf den Prüfling (26) trifft, und

- Berechnen des Intensitätskorrekturparameters (k) aus einer Verschiebung eines Schattenbilds (S, S') des Prüflings (26) auf dem Flachbilddetektor (14) in Abhängigkeit von der Intensität (I) der Röntgenstrahlung.

6. Verfahren nach Anspruch 5, **dadurch gekennzeichnet, dass**

- die Röntgenstrahlung hergestellt wird durch Bestrahlen eines Quellpunkts (Q) eines Targets (20) mit Elektronen, so dass eine Roh-Strahlung entsteht, und
- Filtern der Roh-Strahlung mittels des Filters (24), wobei das Filter höchstens 75% der Gesamt-Intensität der Roh-Strahlung herausfiltert.

7. Röntgen-Computertomograph (10) mit

einer Röntgenquelle (12) zum Erzeugen von Röntgenstrahlung
einem Flachbilddetektor (14), der eine Vielzahl an Pixeln (P) aufweist, zum Messen von pixelabhängigen Intensitätsdaten (I(P)) der Röntgenstrahlung,
einer Bewegungsvorrichtung (32), insbesondere einer Drehvorrichtung zum Bewegen eines Prüflings (26) relativ zur Röntgenquelle (12) und zum Flachbilddetektor (14), und
einer Auswerteeinheit (40) zum Berechnen eines dreidimensionalen Bilds des Prüflings (26) aus den pixelabhängigen Intensitätsdaten (I(P)),
**dadurch gekennzeichnet, dass**
die Auswerteeinheit (40) ausgebildet ist zum automatischen Durchführen eines Verfahrens nach einem der vorstehenden Ansprüche.

8. Röntgen-Computertomograph nach Anspruch 7, **dadurch gekennzeichnet, dass** die Auswerteeinheit (40) ausgebildet ist zum: Erhalten der korrigierten pixelabhängigen Intensitätsdaten (I'(P)) und Berechnen des dreidimensionalen Bilds aus den korrigierten pixelabhängigen Intensitätsdaten (I'(P)).

## Claims

1. A method for dimensional X-ray measurement, in particular by way of computed tomography, featuring the steps:

a) irradiating a test object (26) with non-monochromatic X-ray radiation from a virtually punctiform X-ray source (12),
b) measuring the intensity (I) of the X-ray radiation (22) in the radiation path behind the test object (26) by means of a flat panel detector (14) which has a plurality of pixels (P) to obtain pixel-dependent intensity data (I(P)), and
c) calculating at least one dimension (H) of the test object (26) using the pixel-dependent intensity data (I(P)),
**characterised in that**
d) the pixel-dependent intensity data (I(P)) is corrected by the influence

- of an effective penetration depth ($\tau$) in the flat panel detector (14) caused by beam hardening and/or
- a displacement of the effective source location (Q) on a target (20) of the X-ray source (12) caused by beam hardening is corrected
and
the correction comprises the following steps:
for at least a majority of the pixels (P)

(i) identifying a zero point distance (r) of the pixel (P) from an optical axis (A),
(ii) identifying the intensity (I) of the X-ray radiation measured by the pixel,
(iii) allocating a corrected position (K') depending on the zero point distance (r) and the intensity (I), and
(iv) calculating corrected pixel-dependent intensity data (I') from all corrected positions (K') and the corresponding intensities (I'(P)).

2. The method according to claim 1, **characterised in that** the corrected position (K'), the zero point (N) and the original position (K) lie on one line.

3. The method according to claim 1 or 2, **characterised by** the fact that

a differential distance ($_\Delta$r) between the zero point distance (r') of the corrected position (K') and the zero point distance (r) of the uncorrected position (K) is calculated from
a term (c+k f(I)), which contains a product (k f(I)) of a function (f) of the intensity (I), an intensity correction parameter (k) and a constant (c).

4. The method according to one of the preceding claims, **characterised in that** the X-ray radiation is generated by irradiating a source point (Q) of a target (20) with electrons,

the calculation of the dimensions (H) of the test object (26) is executed using the enlargement factor ($\alpha$), which depends on

a distance (b) of the source point (Q) from the flat panel detector (14) and
a distance (a) of the source point (Q) from the test object (26), and that

the enlargement factor ($\alpha$) is corrected by the influence of an X-ray emission spectrum that changes due to a changing electron penetration depth into the target (20).

5. The method according to claim 3, **characterised by** the steps:

- measuring a test object (26) in the form of a calibration body,
- changing a filter strength (d) of a filter (24) with which the X-ray radiation is filtered before it strikes the test object (26), and
- calculating the intensity correction parameter (k) from a displacement of a shadow image (S, S') of the test object (26) on the flat panel detector (14), depending on the intensity (I) of the X-ray radiation.

6. The method according to claim 5, **characterised in that**

- the X-ray radiation is generated by irradiating a source point (Q) of a target (20) with electrons such that raw radiation occurs, and
- a filtering of the raw radiation by means of the filter (24) takes place, said filter filtering out a maximum of 75 % of the total intensity of the raw radiation.

7. An X-ray computed tomography scanner (10) with

an X-ray source (12) for generating X-ray radiation,
a flat panel detector (14), which features a plurality of pixels (P), for measuring pixel-dependent intensity data (I(P)) of the X-ray radiation,
a movement device (32), especially a rotation device, for moving a test object (26) relative to the X-ray source (12) and the flat panel detector (14), and
an evaluation unit (40) for calculating a three-dimensional image of the test object (26) using the pixel-dependent intensity data (I(P)),
**characterised in that**
the evaluation unit (40) is designed to automatically execute a method according to one of the above claims.

8. The X-ray computed tomography scanner according to claim 7, **characterised by** the fact that the evaluation unit (40) is designed to:

obtain the corrected pixel-dependent intensity data (I'(P)) and
calculate the three-dimensional image using the corrected pixel-independent intensity data (I'(P)).

**Revendications**

1. Procédé de mesure dimensionnelle radiographique aux rayons X, en particulier au moyen de la tomographie assistée par ordinateur, comprenant les étapes consistant à :

a) irradier une éprouvette (26) avec un rayonnement X non monochromatique provenant d'une source de rayons X quasi-ponctuelle (12),

b) mesurer l'intensité (I) du rayonnement X (22) dans le trajet de rayonnement derrière l'éprouvette (26) au moyen d'un capteur plan (14) qui comprend une multitude de pixels (P), de manière à obtenir des données d'intensité (I(P)) dépendant des pixels, et

c) calculer au moins une dimension (H) de l'éprouvette (26) à l'aide des données d'intensité (I(P)) dépendant des pixels,

**caractérisé en ce que**

d) les données d'intensité (I(P)) dépendant des pixels sont corrigées de l'influence

- d'une modification, due au durcissement du faisceau, d'une profondeur de pénétration effective (τ) dans le capteur plan (14), et/ou
- d'un déplacement, dû au durcissement du faisceau, du site source effectif (Q) sur une cible (20) de la source de rayons X (12),

et la correction comprend les étapes suivantes consistant à :

pour au moins une pluralité des pixels (P)

i) déterminer une distance de point zéro (r) du pixel (P) par rapport à un axe optique (A),

ii) déterminer l'intensité (I) du rayonnement X mesuré par le pixel,

iii) attribuer une position corrigée (K') qui dépend de la distance de point zéro (r) et de l'intensité (I), et

iv) à partir de toutes les positions corrigées (K') et des intensités associées (I'), calculer des données d'intensité corrigées (I'(P)) dépendant des pixels.

2. Procédé selon la revendication 1, **caractérisé en ce que** la position corrigée (K'), le point zéro (N) et la position initiale (K) sont situés sur une même ligne.

3. Procédé selon la revendication 1 ou 2, **caractérisé en ce que** une distance différentielle (Δr) entre la distance de point zéro (r') de la position corrigée (K') et la distance de point zéro (r) de la position non corrigée (K) est calculée à partir d'un terme (c+k f(I)) comprenant un produit (k f(I)) d'une fonction (f) de l'intensité (I), d'un paramètre de correction d'intensité (k) et d'une constante (c).

4. Procédé selon l'une des revendications précédentes, **caractérisé en ce que**

le rayonnement X est généré en irradiant un point source (Q) d'une cible (20) avec des électrons,

le calcul de la dimension (H) de l'éprouvette (26) est effectué à l'aide d'un facteur d'agrandissement (α) qui dépend

d'une distance (b) du point source (Q) par rapport au capteur plan (14) et

d'une distance (a) du point source (Q) par rapport à l'éprouvette (26),

et **en ce que** le facteur d'agrandissement (α) est corrigé de l'influence d'un spectre d'émission de rayons X qui varie en raison d'une variation de la profondeur de pénétration des électrons dans la cible (20).

5. Procédé selon la revendication 3, **caractérisé par** les étapes consistant à :

- mesurer une éprouvette (26) sous la forme d'un corps de calibrage,
- modifier l'épaisseur (d) d'un filtre (24) par lequel le rayonnement X est filtré avant de tomber sur l'éprouvette (26), et
- calculer le paramètre de correction d'intensité (k) à partir d'un déplacement d'une image d'ombre (S, S') de l'éprouvette (26) sur le capteur plan (14) en fonction de l'intensité (I) du rayonnement X.

6. Procédé selon la revendication 5, **caractérisé en ce que**

- le rayonnement X est généré en irradiant un point source (Q) d'une cible (20) avec des électrons de manière à générer un rayonnement brut, et
- filtrer le rayonnement brut au moyen du filtre (24), le filtre filtrant au maximum 75 % de l'intensité totale du rayonnement brut.

7. Tomodensitomètre à rayons X (10) comprenant

une source de rayons X (12) pour générer un rayonnement X,

un capteur plan (14) comprenant une multitude de pixels (P) pour mesurer les données d'intensité (I(P)), dépendantes des pixels, du rayonnement X,

un dispositif de déplacement (32), en particulier un dispositif de rotation, pour déplacer une éprouvette (26) par rapport à la source de rayons X (12) et au capteur plan (14), et

une unité d'évaluation (40) pour calculer une image tridimensionnelle de l'éprouvette (26) à partir des données d'intensité (I(P)) dépendant des pixels,

**caractérisé en ce que**

l'unité d'évaluation (40) est réalisée pour mettre en oeuvre automatiquement un procédé selon l'une des revendications précédentes.

8. Tomodensitomètre à rayons X selon la revendication 7, **caractérisé en ce que** l'unité d'évaluation (40) est réalisée pour :

obtenir les données d'intensité corrigées (I'(P)) dépendant des pixels et

calculer l'image tridimensionnelle à partir des données d'intensité corrigées (I'(P)) dépendant des pixels.

Fig. 1

$P_{3,3}$

10

$P_{2,3}$

$r_{2,3}$

$P_{2,2}$

$L_{2,3}$

$P_{1,1}$

y

x

A  N

Fig. 2a

$P_{3,3}$

$\Delta r$

B

$r'_{2,3}$

$P_{2,3}$

$L_{2,3}$

y

x

A  N

Fig. 2b

Fig. 2

Abhängigkeit der Vergrößerung von der Filterung
Prüfling AL-Folie, We = 150 keV

$1,5 \cdot 10^{-4}$

Vergrößerung

Intensität

Aluminiumfilter
Kupferfilter

Fig. 3a

EP 3 577 626 B1

Fig. 3b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8208603 B2

**In der Beschreibung aufgeführte Nicht-Patentliteratur**

- **WEISS et al.** *Proceedings Industrielle Computertomografie Tagung, Wels, Österreich,* 2010

- **VON SHARMA et al.** *Nuclear Instruments and Methods in Physics Research A,* 2017, vol. 841, 117-123